# EUROPEAN PATENT APPLICATION

(11) **EP 2 722 057 A2**
(43) Date of publication of application: **23.04.2014**
(21) Application number: 13150173.6
(22) Date of filing: 03.01.2013
(51) Int. Cl.: A61K 41/00

(54) **A series of drugs using photofrin to catalyze decomposition of hydrogen peroxide**

(30) Priority: 16.10.2012 CN 201210390725
(71) Applicant: Beijing Top Grade-Kang Ming Medical Devices Inc., 100176 Beijing (CN)
(72) Inventor: Zeng, Jun, 100176 Beijing (CN); Sun, Qiyin, 100176 Beijing (CN)
(74) Representative: Meyer-Dulheuer, Karl-Hermann

(57) **Abstract**

Traditional photodynamic therapy (PDT) is a method of using a visible light with a certain wavelength to irradiate photofrin, converting O₂ into ¹O₂ by energy conversion for treatment of diseases, and many photofrin kinds of new drugs have been marketed and widely used in clinic. The present invention relates to therapeutical mechanism totally different from PDT, in which the specific affinity of photofrin to focus, such as tumors, vascular plaques and skin diseases, is utilized, and a certain means is used to activate photofrin in the focus, catalyze the decomposition reaction of H₂O₂ to generate ¹O₂ in the focus, ¹O₂ further induces apoptosis and necrosis of cells with pathological changes, the purpose of treating tumors, vascular plaques and skin diseases is achieved, and cosmetic effects on skin are prompted. A series of new drugs obtained via the screening and studying of the present invention are used for Chemodynamic Therapy (CDT), or for Radiochemodynamic Therapy (RCDT) carried out via radioactive rays. The action mechanism of the drugs is as follows:

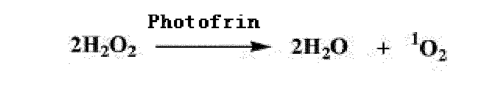

In the formula, photofrin can be endogenous (e.g., 5-ALA synthetic endogenous porphyrin), or exogenous; H₂O₂ can be exogenous, or can be endogenous H₂O₂ generated by means of hydrogen peroxidase inhibitor; the activation of photofrin is carried out by specific protein binding or by electron beam, x-ray, r-ray, or other means. In comparison with traditional photodynamic therapy (PDT), CDT and RCDT have advantages in: no need for light source; no need for oxygen; able to be quantitated; no need for intervention means; good therapeutical effects; simpler operation; capable of being repeated therapy; easy for popularization and application. The present invention relates to a series of new drugs useful in CDT and RCDT. The series of new drugs refer to chemical series capable of catalyzing the decomposition of H₂O₂ to generate singlet oxygen (¹O₂). They have no notable side effects on human body, at same time have affinity to focus of disease. Under this kind of therapeutic model, photofrin acts as a catalyst for chemical reaction, its mechanism, use and therapeutical effects are totally different from the traditional photodynamic therapy, unnecessary for oxygen and visible light, and form an independent series of new drug. The present patent is to protect the intellectual property of the series of drugs.

## Description

### FIELD OF THE INVENTION

The present invention relates to a series of drugs using a photofrin to catalyze in vivo decomposition of hydrogen peroxide (H₂O₂) to generate singlet oxygen (¹O₂) for treatment of diseases or for cosmetology, which can be used for Chemodynamic Therapy (CDT), or used for Radiochemodynamic Therapy (RCDT), "Radiodynamic Therapy (RDT)" in which radioactive rays directly activate photofrin to fulfill the treatment.

### BACKGROUND OF THE INVENTION

In traditional photodynamic therapy (PDT), the specific affinity of photofrin to focus, such as tumors, vascular plaques, skin diseases, is utilized, then a visible light with a specific wavelength capable of being absorbed by photofrin is used to irradiate the focus, after the focus absorbs the light energy, O₂ converts into ¹O₂, and ¹O₂ further induce apoptosis or necrosis of cells with pathological changes, so as to achieve therapeutical effects. However, PDT usually could not achieve desired effects in clinic due to the poor penetrability of visible light, difficulty in therapeutical quantitation, and the deficiency of O₂. In the same time, traumatic or non-traumatic intervention means have to be used to introduce light source to the diseased regions in many situations, so that both doctors and patients feel difficulty to accept PDT.

In order to overcome the drawbacks of PDT, PDT should be modified and improved in abstracto. It is known H₂O₂ can be catalytically decomposed under weak alkaline condition, the reaction almost stoichiometrically produce ¹O₂, the reaction formula is as follows:

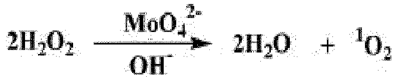

However, this reaction is of low efficiency, uncontrollable, poor specificity to focus, unable to be used in clinic treatment of diseases. In order to use this chemical reaction model in clinic, the following five basic requirements have to be met: 1) the reaction must be performed at focus of disease; 2) the reaction must be carried out in high efficiency; 3) the reaction must be controllable; 4) the reaction must be quantitative; 5) the catalyst should not directly participate in the reaction and no new compound is generated. Hence, the present invention develops a series of creative new drugs of chemical reactions catalyzed by photofrin on the basis of proposal and discussion of the following assumptions, which can decompose H₂O₂ to generate singlet oxygen (¹O₂) without using oxygen and visible light. These series drugs are useful in Chemodynamic Therapy (CDT) and Radiochemodynamic Therapy (RCDT).

### Assumptions for CDT and RCDT

It is assumed that photofrin can catalyze the decomposition reaction of H₂O₂ to generate ¹O₂ with high efficiency under certain condition, and the decomposition reaction of H₂O₂ catalyzed by photofrin is limited to focus of diseases due to the specific affinity of photofrin to focus, such as tumors, vascular plaques, skin diseases.

It is assumed that photofrin can be activated by physical and/or chemical binding energy to prompt the decomposition reaction of H₂O₂ catalyzed by photofrin to generate ¹O₂ with higher efficiency, so that when specific means (such as radiation, biochemistry, heating, etc.) is applied to quantitatively generate ¹O₂ at site of focus, the decomposition reaction of H₂O₂ would be further limited to the focus.

It is assumed photofrin is a key factor for catalyzing the decomposition reaction of H₂O₂ to generate ¹O₂, and the decomposition reaction of H₂O₂ can rapidly start or stop by changing conformation or energy level.

It is assumed in the presence of an amount of photofrin and H₂O₂, its physical quantity of stoichiometric amount is in proportion with the generated ¹O₂. Hence, the irradiation dose of electron beam, x-ray, r-ray and ion beam is in proportion with the measurable amount of the generated ¹O₂, so that the quantitative determination of RCDT can be fulfilled.

It is assumed photofrin only acts as catalyst to catalyze the decomposition reaction of H₂O₂ to generate ¹O₂. Photofrin does not directly participate in the reaction and does not generate new derivatives of photofrin.

### Experimental demonstration of CDT and RCDT

Under weak alkaline condition, the catalytic decomposition reaction of H₂O₂ has a relatively low efficiency even in the presence of ·OH free radicals, the reaction formula is as follows:

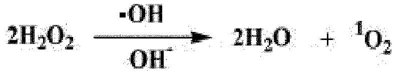

When the content of H₂O₂ is 0.15%, the generation of ¹O₂ free radicals cannot be detected after reaction for 6 h.

When photofrin is added under weak alkaline condition, the reaction rate increases significantly:

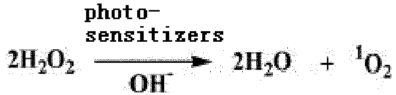

However, the generation of a large amount of ¹O₂ is detected until the concentration of H₂O₂ is 1% or more, and the reaction is performed for 8 h. This reaction can hardly be carried in vivo environment, because: 1) such a high concentration of H₂O₂ cannot be maintained in vivo for a long term; 2) hydrogen peroxidase can rapidly eliminated H₂O₂ in vivo; 3) there is not an alkaline environment in vivo.

When photofrin is activated by radiation or protein binding simultaneously, it can catalyze the decomposition reaction of H₂O₂ rapidly with high efficiency:

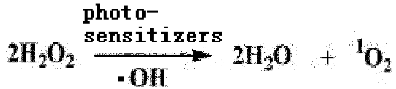

When the concentration of H₂O₂ is 0.03% or less, ¹O₂ can be formed quickly.

X-ray irradiation can electrolyze H₂O to generate ·OH, or the generation of ·OH can be achieved by adding metal ions and Vit-C, which can rapidly start the decomposition reaction of H₂O₂ to generate ¹O₂; once X-ray irradiation stops, or ·OH free radical scavenger is added, the decomposition reaction of H₂O₂ will stop quickly.

When the contents of photofrin and H₂O₂ are fixed, the irradiation dose of X-ray is in proportion with the yield of ¹O₂.

Fluorescence spectrophotometer is used to analyze the structure of photofrin before and after being used to catalyze the decomposition reaction of H₂O₂, it is found that the content and structure of photofrin do not change before and after the reaction.

The above important findings bring the theory of Chemodynamic Therapy (CDT) and Radiochemodynamic Therapy (RCDT), which is the basis for developing these series drugs.

Photofrin is used as catalyst for decomposing H₂O₂ in vivo to finally generate ¹O₂, and in the present invention, this therapeutic model is called as Chemodynamic Therapy (CDT), while the CDT carried out by radioactive rays is called as Radiochemodynamic Therapy (RCDT). Both CDT and RCDT are promising in overcoming the drawbacks of traditional photodynamic therapy (PDT), especially when combining to radiotherapy or pharmacotherapy, ¹O₂ therapeutic model is more suitable in clinical application.

At this time, the mechanism, use and therapeutic effects of photofrin are totally different from those of traditional photodynamic therapy, thus an independent series of new drugs are developed for use in combination with H₂O₂, and activation via hydroxyl free radicals. The present invention is to seek intellectual property protection for the series of drugs.

### Advantages of CDT and RCDT

**No need for light source:** in traditional photodynamic therapy (PDT), light source is the most important factor, the quality of light source directly determine the therapeutical effects. In addition, photofrin used for PDT should be designed according to light source to maximize the absorption of light, and for lights with different wavelengths, the used photofrin should have different chemical structure. CDT and RCDT do not need light source. In the presence of an amount of photofrin, electron beam, x-ray, r-ray or ion beam, or protein binding can activate the photofrin, start the decomposition reaction of H₂O₂ to generate ¹O₂. The therapeutical effect of photofrin is not determined by the ability of absorbing light, but determined by the ability of catalyzing the decomposition of H₂O₂.

**No need for oxygen:** many tumors are at O₂ deficient state, while the reaction substrate of traditional PDT is O₂, i.e., when there is not oxygen, ¹O₂ cannot be generated and therapeutical effect cannot be achieved. CDT and RCDT do not need O₂, their reaction substrate is H₂O₂, while a low concentration of exogenous or endogenous H₂O₂ would meet the therapeutical requirement.

**Good therapeutical effects:** (1) Visible light has poor tissue penetrability, and poor therapeutical effect in PDT for large focus or deep focus. CDT and RCDT do not need light source, so that CDT and RCDT can act in whole range of focus of disease whatever the size of depth of focus. (2) In therapeutical procedure of PDT, photofrin content decreases rapidly, and therapeutical effect becomes weak accordingly. CDT and RCDT use photofrin as reaction catalyst, and the structure of photofrin would not be destroyed and its content would not be reduced during the therapeutical procedure, so that therapeutical effects are maintained. (3) CDT and RCDT generate ·OH and ¹O₂ at the same time, increasing the killing effects of free radicals. (4) When electron beam, X-ray, r-ray or ion beam are used to carry out RCDT, these radioactive rays per se have function of killing cells with pathological changes, thereby bring about double effects. (5) When electron beam, X-ray, r-ray or ion beam are used to carry out RCDT, target regions can be designed by three-dimensional stereotaxis, together with the specific affinity of photofrin to target regions, forming double target effects, which not only improves therapeutical effects, but also reduces side-effects. (6) photofrin per se has a certain effects on enhancing sensitivity in radiotherapy. (7) H₂O₂ can also be decomposed by hydrogen peroxidase in tumors to generate O₂, thereby improving oxygen deficient condition of tumors and enhancing therapeutical effects.

**Less side-effects:** Since two conditions, presence of photofrin and activation of photofrin, are required to start decomposition reaction of H₂O₂ to generate ¹O₂, photofrin is concentrated at focus and activated in target region of focus, so that the decomposition reaction of H₂O₂ is limited in the region of focus, and rarely occurs at sites around focus and at other sites of body, wherein the decomposition reaction of H₂O₂ even does not occur. In addition, H₂O₂ can be rapidly decomposed in vivo by hydrogen peroxidase to form H₂O and O₂.

**Capable of being quantitated:** One of the serious drawbacks of traditional PDT is difficulty in determining therapeutical dose, which directly influences therapeutical effects and evaluation thereof. RCDT can totally fulfill quantitation, because the amount of ¹O₂ generated in RCDT is in proportion with the irradiation dose of x-ray, r-ray and so on. With the great progress in current precise radiotherapy, precise RCDT can be fully fulfilled.

**No need for intervention means:** Since visible light has poor tissue penetrability, traumatic or non-traumatic intervention means are usually used in traditional PDT to introduce light source into focus of disease. These means may not be accepted by many doctors and patients, so that many patients lose an opportunity to be treated. Since x-ray, r-ray and the like have a tissue penetrability significantly higher than visible light and can reach any sites of body, RCDT does not need any intervention means.

**Simple operation:** Even for superficial lesions, traditional PDT still requires light source to irradiate focus, which operation is complex and difficult in popularization. CDT is very simple to therapy superficial lesions, comprising separately coating photofrin, H₂O₂ or H₂O₂-containing preparation in order on the lesions, directly activating photofrin to start decomposition reaction of H₂O₂, generating ¹O₂. At the end of therapy, the activation of photofrin stops, to terminate the decomposition reaction of H₂O₂. In combination with radiotherapy, the relevant technology is well developed, and the operations thereof are highly automatized.

**Capable of being continuous and repeated therapy:** Traditional PDT destroys photofrin, usually requires interventional means, and thus can hardly be used for continuous and repeated therapy. However, continuous and repeated therapy is very important in many conditions, for example, for patients with tumors, tumor vessels are targets for immediate therapy after intravenous injection of photofrin, and tumor cells are targets for delay therapy. CDT and RCDT do not destroy photofrin, do not need intervention means, and thus are competent enough for segmented repeated therapeutic protocols.

**Easy for popularization and application:** Due to the above advantages, CDT and RCDT are easier for popularization and application in clinic.

### SUMMARY OF THE INVENTION

### Mechanism of CDT and RCDT

Traditional photodynamic therapy (PDT) is a method of using a visible light with certain wavelength to irradiate photofrin, converting O₂ into ¹O₂ by energy conversion to treat diseases.

The present invention relates to therapeutical mechanism, means and series drugs totally different from PDT, in which the specific affinity of photofrin to focus, such as tumors, vascular plaques and skin diseases, is utilized, and a certain means is used to activate photofrin in the focus, rapidly start (or stop) the decomposition reaction of H₂O₂ to generate ¹O₂ in the focus, ¹O₂ further induces apoptosis and necrosis of cells with pathological changes, the purpose of treating tumors, vascular plaques and skin diseases is achieved, and cosmetic effects on skin are prompted. On this theoretical basis, the present invention develops a series of new drugs for this kind of therapeutical model. These drugs can be used for Chemodynamic Therapy (CDT), or be used for CDT carried out via radioactive rays, which is so-called as Radiochemodynamic Therapy (RCDT). The action mechanism of the drugs is as follows:

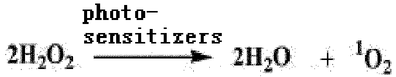

In the formula, photofrin can be endogenous (e.g., 5-ALA synthetic endogenous porphyrin), or exogenous; H₂O₂ can be exogenous, or can be endogenous H₂O₂ generated by means of hydrogen peroxidase inhibitor; the activation of photofrin is carried out by specific protein binding or by electron beam, x-ray, r-ray, or other means.

The series of drugs of the present invention can overcome the drawbacks of photodynamic therapy (PDT), do not use visible light source, do not rely on oxygen.

### Photofrin catalysts used for CDT and RCDT

Different from the purpose and function of photofrin used in PDT, PDT uses photofrin to perform photo energy conversion, while CDT and RCDT use photofrin as catalyst for decomposition reaction of H₂O₂. Under certain physical and chemical conditions, this kind of photofrin has high efficiency in catalyzing the decomposition reaction of H₂O₂, to rapidly generate ¹O₂.

Different from the action effects of photofrin used in PDT, the photofrin used in PDT should be designed according to light source to maximize absorption of light, and as for lights with different wavelengths, the chemical structure of the used photofrin should be different. The therapeutical effects of photofrin in CDT and RCDT do not depend on its absorption of light, while depend on its ability of catalyzing decomposition of H₂O₂. The photofrin having good therapeutical effects in PDT may not catalyze the decomposition reaction of H₂O₂ with high performance, and thus may not be suitable for CDT and RCDT, vice versa.

Different from the chemical structure of photofrin used in PDT, the photofrin used in PDT is designed and developed according to the absorptivity of visible light with certain wavelengths, while the photofrin used in CDT and RCDT is designed and developed according to the catalytic efficiency for decomposition reaction of H₂O₂

Different from the fate of photofrin used in PDT, the photofrin is destroyed by visible light in PDT, while the photofrin is merely used as reaction catalyst in CDT and RCDT, which chemical structure is not destroyed.

### Methods for researching and developing a series drugs useful in CDT and RCDT

Photofrins with different structures and same molar concentration are taken, and separately mixed with H₂O₂ solutions with different concentrations in vacuum reaction bottles away from light. Then a suitable amount of ¹O₂ free radical indicator (e.g., 9,10-dimethylanthracene, etc.) is added. The decomposition reaction of H₂O₂ starts and stops according to the following methods, and the content of ¹O₂ free radicals is finally measured directly or indirectly by a fluorescence spectrophotometer.

The mixture solutions of photofrin and H₂O₂ in vacuum reaction bottles away from light are irradiated by quantitative (Gy) x-ray, r-ray or other methods, to start the decomposition reaction of H₂O₂ catalyzed by photofrin, and the decomposition reaction of H₂O₂ catalyzed by photofrin is terminated by stopping the irradiation. ¹O₂ can be quantitatively analyzed.

A photofrin-binding protein in a quantitative amount is added to the vacuum reaction bottles containing a mixture solution of photofrin and H₂O₂ away from light, to start the decomposition reaction of H₂O₂ catalyzed by photofrin, and the decomposition reaction of H₂O₂ catalyzed by photofrin is terminated by destroying protein function (e.g., by ethanol).

Thus, photofrin catalysts with high performance can be screened out.

### Experimental methods for CDT and RCDT

### Cell tests

Various kinds of cells are cultivated, for performing relevant researches of CDT and RCDT.

### Tumor models and therapeutical researches

Various tumor-bearing animal models are prepared, for performing treatment of tumors by CDT and RCDT, treatment of tumor vessels by CDT and RCDT, and immunotherapy researches relevant to tumors by CDT and RCDT.

### Vascular plaques and therapeutical researches

Various animal models with vascular injury or vascular plaques are prepared, for performing treatment of vascular plaques by CDT and RCDT, including treatment of plaques of small vessels (eye ground vessels) and large vessels (aorta) by CDT and RCDT.

### Other animal models and therapeutical researches

Various animal models with skin diseases are prepared, for performing local treatment by CDT and RCDT.

### Treatment of tumors by series of drugs for RCDT

**Step 1,** positioning tumors by images, completing the sketch of RCDT tumor targets and the protocol of RCDT by radio-therapeutical planning system;
**Step 2,** administering photofrin or photofrin prodrug (e.g., 5-ALA) systematically or topically to tumor part, utilizing the specific affinity of photofrin to tumor tissues to concentrate photofrin at tumor cells;
**Step 3,** administering exogenous H₂O₂ (e.g., injection of carbamide peroxide, or H₂O₂) systematically or topically to tumor part, or using hydrogen peroxidase inhibitor to generate endogenous H₂O₂ in vivo;
**Step 4,** after administering exogenous H₂O₂ systematically or topically to tumor part, using an instrument for radiotherapy, immediately carrying out the RCDT plan, using electron beam, x-ray, r-ray or ion beam to activate photofrin to catalyze the decomposition reaction of H₂O₂ to generate ¹O₂. The administration of H₂O₂ is for early treatment by killing tumor vessels, and for delay treatment by killing tumor cells;
**Step 5,** stopping the irradiation of electron beam, x-ray, r-ray or ion beam, thus immediately terminating the decomposition reaction of H₂O₂, the residual H₂O₂ can be decomposed by hydrogen peroxidase to form harmless H₂O and O₂. If necessary, RCDT can be repeatedly carried out according to a reasonable time schedule.

### Treatment of tumor vessels by series of drugs for RCDT

**Step 1,** positioning tumors by images, completing the sketch of RCDT tumor targets and the protocol of RCDT by radio-therapeutical planning system;
**Step 2,** preparing fresh photofrin and exogenous H₂O₂ or H₂O₂-containing preparations;
**Step 3,** administering photofrin and exogenous H₂O₂ or H₂O₂-containing preparations to tumor topical control vessels or whole body vessels;
**Step 4,** after administering photofrin and exogenous H₂O₂ or H₂O₂-containing preparations, using an instrument for radiotherapy, immediately carrying out the RCDT plan, using electron beam, x-ray, r-ray or ion beam to activate photofrin in vascular endothelial cells of tumor or vascular interstitial tissue to start the decomposition reaction of H₂O₂ catalyzed by photofrin to generate ¹O₂, so as to destroy tumor vessels;
**Step 5,** stopping the irradiation of electron beam, x-ray, r-ray or ion beam, thus immediately terminating the decomposition reaction of H₂O₂, the residual H₂O₂ can be decomposed by hydrogen peroxidase to form harmless H₂O and O₂. If necessary, RCDT can be repeatedly carried out according to a reasonable time schedule.

### Treatment of vascular plaques by series of drugs for RCDT

**Step 1,** positioning vascular plaques by images, completing the sketch of RCDT vascular plaque targets and the protocol of RCDT by radio-therapeutical planning system;
**Step 2,** administering photofrin or photofrin prodrug (e.g., 5-ALA) systematically, utilizing the specific affinity of photofrin to vascular plaques to concentrate photofrin at vascular plaques;
**Step 3,** administering exogenous H₂O₂ (e.g., injection of carbamide peroxide, or H₂O₂) systematically, or using hydrogen peroxidase inhibitor to generate endogenous H₂O₂ in vivo;
**Step 4,** after administering H₂O₂ systematically, using an instrument for radiotherapy, immediately carrying out the RCDT plan, using electron beam, x-ray, r-ray or ion beam to activate photofrin in vascular plaques to start the decomposition reaction of H₂O₂ catalyzed by photofrin to generate ¹O₂, so as to ablating vascular plaques;
**Step 5,** stopping the irradiation of electron beam, x-ray, r-ray or ion beam, thus immediately terminating the decomposition reaction of H₂O₂, the residual H₂O₂ can be decomposed by hydrogen peroxidase to form harmless H₂O and O₂. If necessary, RCDT can be repeatedly carried out according to a reasonable time schedule.

### Treatment of other diseases by series of drugs for RCDT

The therapeutical procedure is similar to the aforementioned, suitable for topical disease in deep sites of body, for example, benign diseases such as topical intractable inflammations, ophthalmic diseases, vascular tumors in organs, prostatic hyperplasia, etc.

### Treatment of skin diseases by series of drugs for CDT

It is useful in treatment of all kinds of skin diseases suitable for PDT.
**Step 1,** administering photofrin or photofrin prodrug (e.g., 5-ALA) systematically or topically, utilizing the specific affinity of photofrin to skin lesions to concentrate photofrin at skin lesions;
**Step 2,** administering H₂O₂ or H₂O₂-containing preparations to the skin lesions;
**Step 3,** when administering a specific activator to the skin lesions, starting the decomposition reaction of H₂O₂ catalyzed by photofrin to generate ¹O₂, so as to achieve the purpose of treating skin diseases;
**Step 4,** washing the skin lesions with activator scavenger (e.g., mannitol, ethanol, etc.) to terminate the decomposition reaction of H₂O₂.

This method is simple in operation, needs no light source, and can be repeated as external application drugs.

### Treatment of tumor cells and tumor vessels by series of drug for CDT

An intervention means is required as assistant.
**Step 1,** administering photofrin or photofrin prodrug (e.g., 5-ALA) systematically, utilizing the specific affinity of photofrin to tumor tissues to concentrate photofrin at tumor cells;
**Step 2,** selectively intubating tumor blood supplying vessels, or directly injecting tumor body;
**Step 3,** preparing fresh photofrin and exogenous H₂O₂;
**Step 4,** injecting freshly prepared photofrin and exogenous H₂O₂ into tumor bed separately via vessel cannula, or directly slowly injecting into tumor bed;
**Step 5,** after injection, using a physical or chemical method to activate the decomposition reaction of H₂O₂ catalyzed by photofrin to generate ¹O₂, so as to kill tumor cells and destroy tumor vessels.

### Treatment of other disease by series drugs for CDT

For example, drug-resistant infections, topical intractable inflammations, ophthalmic diseases, vascular tumors in organs, prostatic hyperplasia, etc.

### BRIEF DESCRIPTION OF THE DRAWINGS

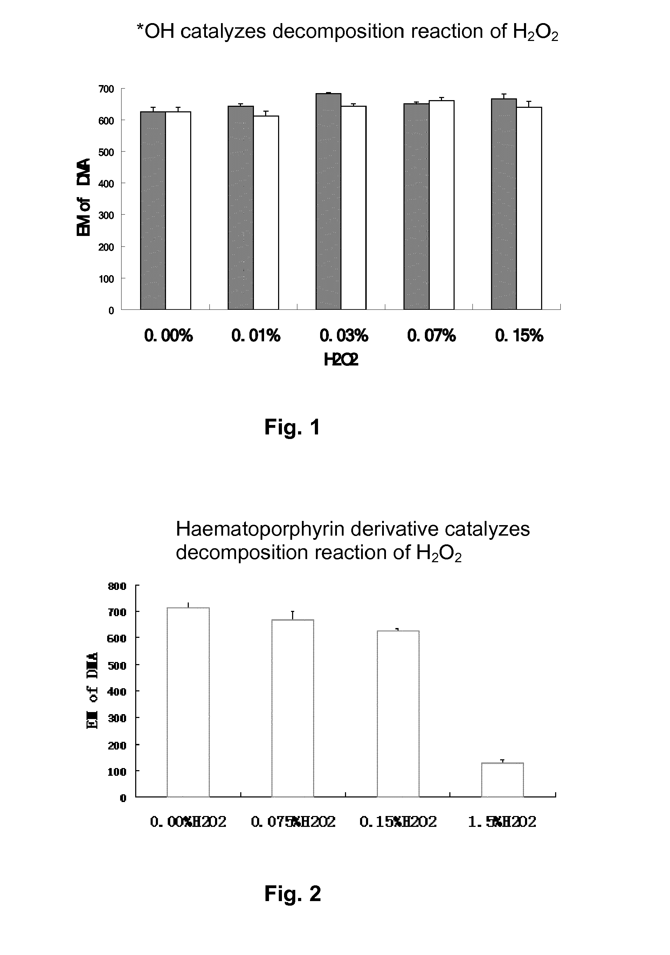

- Fig.1: shows irradiation of 5Gy x-ray in grey histogram, metal ion induction reaction in colorless histogram, the results show when the concentration of H₂O₂ is lower than or equal to 0.15%, both of these two methods do not exhibit obvious generation of ¹O₂.
- Fig.2: shows when the concentration of H₂O₂ is lower than or equal to 0.15%, haematoporphyrin cannot significantly catalyze the decomposition reaction of H₂O₂, to generate ¹O₂. When the concentration of H₂O₂ is 1.5%, haematoporphyrin can slowly catalyze the decomposition reaction of H₂O₂, to generate ¹O₂.

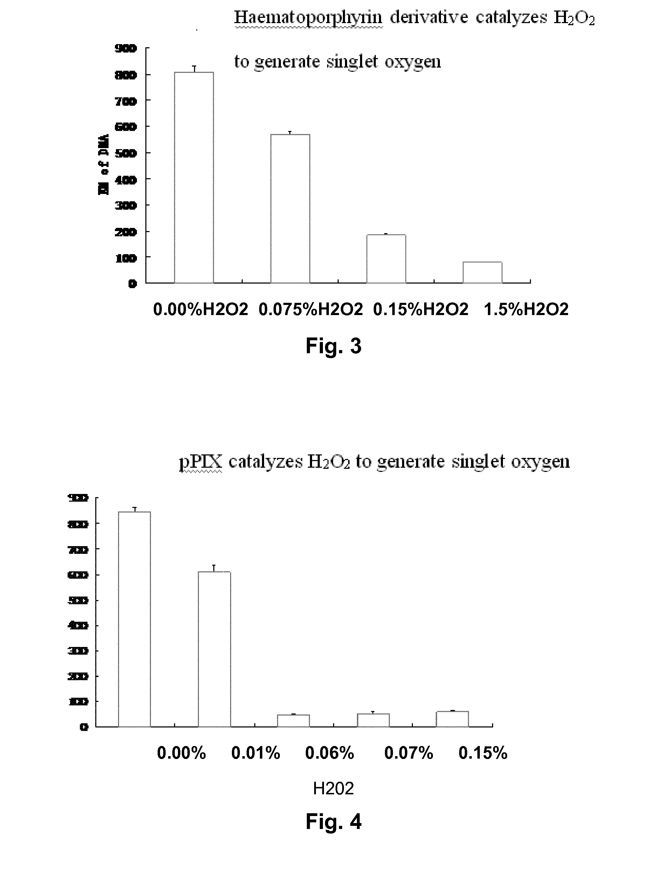

- Fig.3: shows haematoporphyrin can rapidly catalyze the decomposition reaction of H₂O₂, to generate ¹O₂, in comparison with the catalytic reaction using merely haematoporphyrin, the reaction time is shortened from 480 min to about 1 min, the reaction rate is elevated by near 500 times. In addition, the used concentration of H₂O₂ is only 1/20 to 1/50 of that of the catalytic reaction using merely haematoporphyrin, and the catalytic efficiency increases by 20 to 50 times.
- Fig.4: shows an acidified porphyrin PpIX can rapidly catalyze the decomposition reaction of H₂O₂, to generate ¹O₂, in comparison with the catalytic reaction using merely haematoporphyrin, the reaction time is shortened from 480 min to about 1 min, the reaction rate is elevated by near 500 times. In addition, the used concentration of H₂O₂ is only 1/20 to 1/50 of that of the catalytic reaction using merely haematoporphyrin, and the catalytic efficiency increases by 20 to 50 times.

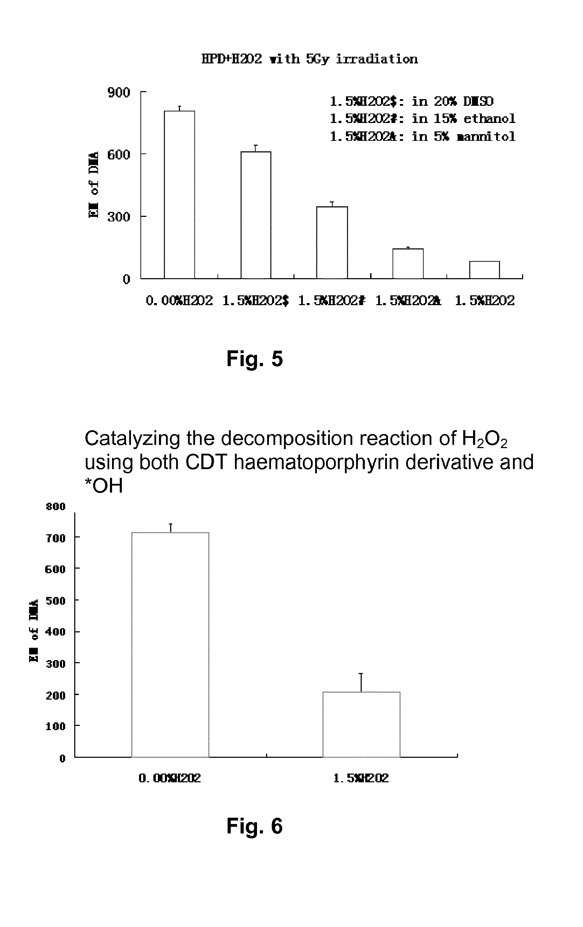

- Fig.5: shows when using DMSO, ethanol and mannitol to eliminate ·OH, the decomposition reaction of H₂O₂ catalyzed by haematoporphyrin is rapidly suppressed, even under condition of high concentration of H₂O₂.
- Fig.6: shows to a mixture solution of haematoporphyrin (15uM) and 1.5% H₂O₂, 0.5mM FeCl3 (FeCl3·6H2O) and 0.5mM Vit-C are added, sufficiently mixed, H₂O₂ is decomposed to generate ·OH, starting the decomposition of H₂O₂ to produce ¹O₂. In comparison with the catalytic reaction catalyzed using only haematoporphyrin, the reaction time is shortened from 480 min to 5 min.

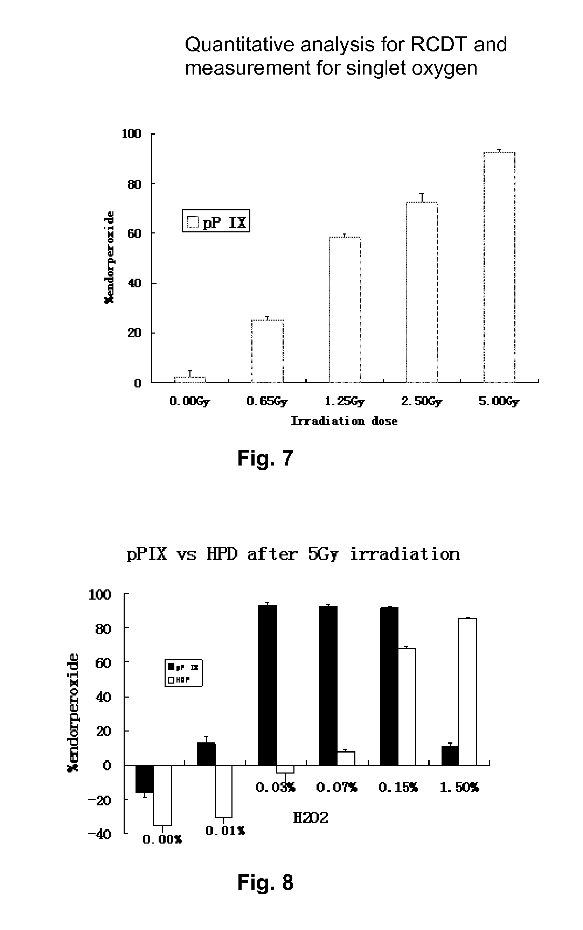

- Fig.7: shows under condition of low concentration of H₂O₂, the contents of photofrin and H₂O₂ are fixed, x-ray is used to start the decomposition reaction of H₂O₂, and the amount of the generated ¹O₂ is in proportion with the irradiation dose.
- Fig.8: shows PpIX in violet histogram, HPD in orange histogram. Under the same conditions, the catalytic effect of PpIX is higher than HPD by 50 times or more. When the concentration of H₂O₂ is 0.03%, PpIX and X-ray catalyze the decomposition reaction of H₂O₂, so that 90% or more of DMA is bound by ¹O₂ free radicals; even when the concentration of H₂O₂ is 0.01 %, PpIX and X-ray can catalyze the decomposition reaction of H₂O₂, so that near 40% (background subtraction) of DMA is bound by ¹O₂ free radicals.

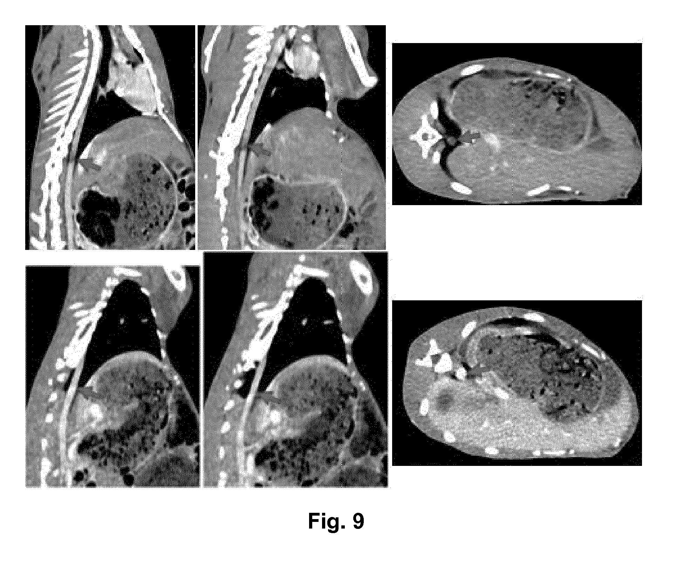

- Fig.9: shows plaques of thoracic aorta of New Zealand rabbit (indicated by upper arrows), in which contrast media is blocked. After radiodynamic therapy for once for one week, the plaques disappear (indicated by lower arrows), in which contrast media pass smoothly.

### DETAILED DESCRIPTION OF THE INVENTION

### Example 1: Weak alkali and ·OH free radical catalyzing the decomposition reaction of H₂O₂

### Experimental methods:

(1) 10ml vacuum bottles were used, and the vacuum bottles were totally sealed with Kodak photographic film light protecting paper.
(2) H₂O₂ solutions (in DMF: H₂O = 1:5) with different concentrations were added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 50MeV X-ray was used to irradiate the vacuum bottles, with dose of 5Gy, to start the decomposition reaction of H₂O₂; the reaction was terminated by stopping x-ray irradiation.
(5) or 0.1mM FeCl₃ (FeCl₃·6H₂O) and 0.1mM Vit-C were added, to start the decomposition of H₂O₂, after decomposition of H₂O₂ for 5 min, 20% was used to terminate the reaction.

### Experimental results:

The results show that when the concentration of H₂O₂ is lower than or equal to 0.15%, whether ionizing H₂O with high energy X-ray or generating ·OH by chemical method cannot reduce DMA fluorescence degree, on the contrary, the DMA fluorescence degree even increases slightly (but showing no statistics significance). This indicates when the concentration of H₂O₂ is relatively low, the decomposition reaction of H₂O₂ cannot be started by using only ·OH, and ¹O₂ free radical cannot be generated (see: Fig.1).

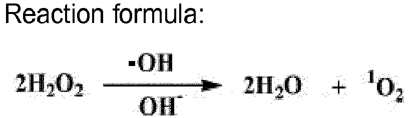

### Example 2: Weak alkali and photofrin catalyzing the decomposition reaction of H₂O₂

### Experimental method:

(1) 10ml vacuum bottles were used, and the vacuum bottles were totally sealed with Kodak photographic film light protecting paper.
(2) H₂O₂ solutions (in DMF: H₂O = 1:5) with different concentrations were added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 15uM Haematoporphyrin derivative was added, and reacted at 25°C for 8 h.

### Experimental results:

The results show that similar to the decomposition reaction of H₂O₂ catalyzed by ·OH, when the concentration of H₂O₂ is lower than or equal to 0.15%, the decomposition reaction of H₂O₂ cannot be catalyzed by haematoporphyrin derivative, to generate ¹O₂. When the concentration increases to 1.5%, haematoporphyrin can catalyze the decomposition reaction of H₂O₂, to generate ¹O₂. This reaction is very slow, needs about 8 h of reaction time, and this reaction can be carried out only under condition of high concentration of H₂O₂ (see: Fig.2).

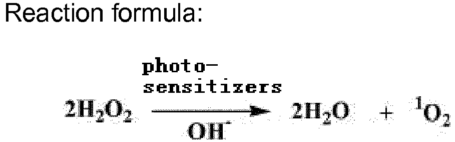

The above 5-1 and 5-2 experimental results show that when merely using ·OH or photofrin as catalyst, the catalytic decomposition reaction of H₂O₂ in vivo, because 1) H₂O₂ concentration cannot be maintained at high level in body for a long time period (8 h); 2) H₂O₂ can be rapidly eliminated in vivo by hydrogen peroxidase; 3) there is not an alkaline environment in body.

### Example 3: Catalyzing the decomposition reaction of H₂O₂ using photofrin and physical method (RCDT method)

### Experimental method:

(1) 10ml vacuum bottles were used, and the vacuum bottles were totally sealed with Kodak photographic film light protecting paper.
(2) H₂O₂ solutions (in DMF: H₂O = 1:5) with different concentrations were added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 15uM Haematoporphyrin derivative (HPD) was added, or 15uM acidified porphyrin (Protoporphyrin IX, PpIX) was added.
(5) The vacuum bottles were irradiated with 10MeV X-ray for about 1 min, dose of 5Gy, to start the decomposition reaction of H₂O₂. The reaction was terminated by stopping irradiation.

### Experimental results:

(1) Haematoporphyrin and x-ray activation could rapidly catalyze the decomposition reaction of H₂O₂, to generate ¹O₂, in comparison with the reaction catalyzed by using merely haematoporphyrin, the reaction time is shortened from 480 min to about 1 min, and the reaction rate increased by near 500 times (see: Fig.3).
(2) Haematoporphyrin and x-ray activation could rapidly catalyze the decomposition reaction of H₂O₂, to generate ¹O₂, the used concentration of H₂O₂ was 1/20 to 1/50 of the concentration (1.5%H₂O₂) used for the reaction catalyzed by merely using haematoporphyrin, the catalytic efficiency increased by 20 to 50 times (see: Fig.3).
(3) P_{P}IX and X-ray activation could rapidly catalyze the decomposition reaction of H₂O₂, to generate ¹O₂, in comparison with the reaction catalyzed by using merely haematoporphyrin, the reaction time is shortened from 480 min to about 1 min, and the reaction rate increased by near 500 times (see: Fig.4).
(4) PpIX and x-ray activation could rapidly catalyze the decomposition reaction of 0.01% H₂O₂, to generate O₂, the used concentration of H₂O₂ was 1/150 of the concentration (1.5%H₂O₂) used for the reaction catalyzed by merely using haematoporphyrin, the catalytic efficiency increased by 150 times (see: Fig.4).
(5) When using 20% DMSO, 15% ethanol, or 5% mannitol to eliminate •OH, the decomposition reaction of H₂O₂ catalyzed by haematoporphyrin and ·OH was rapidly suppressed. Even under condition of high concentration of 1.5% H₂O₂, 20% DMSO, 15% ethanol and 5% mannitol could still inhibit 75.1%, 43.0% and 17.7% of ¹O₂ yield, respectively (see: Fig.5).

The decomposition reaction of H₂O₂ catalyzed by photofrin and •OH together has formula and pharmaceutical mechanism as follows:

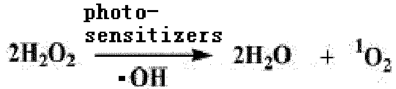

This reaction has the following features: (1) rapid catalyst reaction rate, in comparison with the reaction catalyzed by using merely haematoporphyrin, the reaction rate increases by near 500 times. (2) high catalytic efficiency, in comparison with the reaction catalyzed by using merely haematoporphyrin, even a very low concentration of H₂O₂ (0.01%) can still generate ¹O₂, the catalytic efficiency increased by 150 times. (3) the catalysis of reaction is irrelevant to OH⁻, because PpIX has a higher catalytic efficiency than haematoporphyrin. (4) •OH plays an crucial role in the catalysis reaction, can rapidly start or stop the decomposition reaction of H₂O₂, •OH scavenger can rapidly inhibit the catalysis reaction.

The above results show that both of photofrin and ·OH are necessary conditions for highly efficient catalysis of the decomposition reaction of H₂O₂ to generate ¹O₂. Since the life of •OH is transient (10⁻⁶ s), it can be used for quickly starting and stopping the decomposition reaction of H₂O₂.

### Example 4: Catalyzing the decomposition reaction of H₂O₂ using photofrin and ·OH free radical (CDT method)

### Experimental method:

(1) 10ml vacuum bottles were used, and the vacuum bottles were totally sealed with Kodak photographic film light protecting paper.
(2) H₂O₂ solution (in DMF: H₂O = 1:5) with concentration of 1.5% was added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 15uM Haematoporphyrin derivative was added.
(5) 0.5mM FeCl₃ (FeCl₃·6H₂O) and 0.5mM Vit-C were added, sufficiently mixed, H₂O₂ was decomposed to generate •OH, so as to start the decomposition reaction of H₂O₂ to generate ¹O₂.
(6) It was 5 min after the reaction started, •OH scavenger (20% ethanol) was added, to terminate the reaction, DMA fluorescence degree was measured.

### Experimental results:

In the mixture solution of haematoporphyrin (15uM) and 1.5% H₂O₂, 0.5mM FeCl₃ (FeCl₃·6H₂O) and 0.5mM Vit-C were added, mixed sufficiently, H₂O₂ was decomposed to generate •OH, so as to start the decomposition of H₂O₂ to generate ¹O₂. In comparison with the reaction catalyzed by using merely haematoporphyrin, the reaction time was shortened from 480 min to 5 min. This indicates the ·OH generated by chemical method can also rapidly start the decomposition reaction of H₂O₂ to generate ¹O₂ (see: Fig.6).

### Example 5: Using RCDT method to quantitatively analyze and measure ¹O₂ Experimental method:

(1) 10ml vacuum bottles were used, and the vacuum bottles were totally sealed with Kodak photographic film light protecting paper.
(2) H₂O₂ solution (in DMF: H₂O = 1:5) with concentration of 0.075% was added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 15uM acidified haematoporphyrin (Protoporphyrin IX, PpIX) was added.
(5) X-ray in different doses were used to irradiate the vacuum bottles.

### Experimental results:

Under condition of low concentration of H₂O₂, the contents of photofrin and H₂O₂ were fixed, X-ray started the decomposition reaction of H₂O₂, and the amount of the generated ¹O₂ was in proportion with the irradiation dose. Using EXCEL correlation analysis CORREL function to calculate coefficient correlation of the ¹O₂ amount and the irradiation dose, r = 0.91302, which indicated the correlation between the ¹O₂ amount and the irradiation dose was very significant (see: Fig.7).

The traditional clinical PDT cannot be used for direct quantitative analysis of therapy and cannot measure ¹O₂, which is one of the main factors preventing PDT from clinical application.

The above results show that the contents of photofrin and H₂O₂ were fixed, X-ray started the decomposition reaction of H₂O₂, and the amount of the generated ¹O₂ was in proportion with the irradiation dose. This indicates that the therapeutical dose of RCDT (i.e., ¹O₂ yield) can fully analyzed and measured by using the irradiation dose of electron beam, x-ray, or ion beam.

### Example 6: Screening and studying and developing a series of drugs for CDT or RCDT

### Experimental method:

(1) 10ml vacuum bottles were used, and the vacuum bottles were totally sealed with Kodak photographic film light protecting paper.
(2) H₂O₂ solutions (in DMF: H₂O = 1:5) with different concentrations were added to the sealed vacuum bottles.
(3) 5uM 9,10-dimethylanthracene (DMA) was added. DMA was a kind of ¹O₂ specific indicator (probe), DMA had intensive fluorescence property, after binding to ¹O₂, endorperoxide without fluorescence property was formed, so that the amount of the generated ¹O₂ is in inverse proportion with the content of DMA.
(4) 15uM Haematoporphyrin derivative (HPD), or 15uM acidified porphyrin (Protoporphyrin IX, PpIX) was added.
(5) The vacuum bottles were irradiated with 10MeV X-ray, dose of 5Gy, to start the decomposition reaction of H₂O₂. The reaction was terminated by stopping irradiation.

### Experimental results:

Since PDT is totally different from CDT or RCDT in therapeutical mechanism, a photofrin suitable for PDT may not be suitable for CDT or RCDT. For example, it is found in the present example, the haematoporphyrin widely used in clinical PDT is far less efficient in catalyzing the decomposition reaction of H₂O₂ to generate ¹O₂ than PpIX.

Under the same conditions, the catalytic effect of PpIX is higher by 50 times or more than HPD. When H₂O₂ concentration is 0.01 %, PpIX and X-ray activation can catalyze the decomposition reaction of H₂O₂, so that near 40% (background subtraction) of DMA binds to ¹O₂ free radicals; when the concentration of H₂O₂ is 0.03%, PpIX and X-ray catalyze the decomposition reaction of H₂O₂, so that 90% or more of DMA binds to ¹O₂ free radicals. However, even the concentration of H₂O₂ reaches 1.5%, such effects cannot be achieved by haematoporphyrin and x-ray in catalyzing the decomposition reaction of H₂O₂ (see: Fig.8).

The above results show that photofrins with different structures have different efficiencies in catalyzing the decomposition reaction of H₂O₂, and photofrins with higher efficiency and practicality for CDT and RCDT could be obtained by further studying and developing.

### Example 7: Example of treatment of vascular plaques by RCDT method

### Experimental method:

(1) Male New Zealand rabbits, bodyweight 3-4 kg, were fed with diet containing 1% cholesterol and pork fat, raised for 11 weeks, until atherosclerotic plaques occurred at vessels of eye ground at both sides and artery.
(2) The rabbits were anesthetized with ketamine and thiopental sodium.
(3) Target regions of plaques at artery to be treated were determined by total body CT plain scanning and CTA enhancing.
(4) Target regions of plaques at artery to be treated were sketched by a therapy planning system, to formulate a therapeutical plan suitable for **RCDT,** using GTV dose of up to 5.0Gy, irradiated once. Normal feeding was restored during the procedure.
(5) It was 2 h before the irradiation that 5-ALA (80mg/kg in saline 5ml) was intraperitoneally injected, carbamide peroxide (injection containing H₂O₂, 80mg/kg) in 5ml of saline was slowly injected via ear vein immediately before the irradiation, and the therapeutical plan was carried out immediately after the injection of carbamide peroxide.
(6) It was 5-7 days after irradiation the CTA was reexamined, and the situations of elimination of vascular plaques were recorded.

The New Zealand rabbit models of vascular plaques are common animal models. New Zealand rabbits are fed with high-cholesterol for 3 months, and CTA visualization and MR show the formation of plaques in thoracic aorta. The plaques are used as target regions, positioned, and radiotherapy plan is formulated. It is 1.5 h after intraperitoneal injection of 5-ALA (80 mg/kg of body weight) that a substrate is intravenously dripped, the vascular plaques are irradiated with 5Gy once. The New Zealand rabbits are continuously fed for one week. On the 7^{th} day after therapy, CTA visualization and MR examination are performed again, and the results show that the plaques in thoracic aorta completely disappeared (see: Fig.9).

## Claims

1. A drug prepared on the basis of using a photofrin to catalyze in vivo decomposition of hydrogen peroxide to generate singlet ¹O₂, **characterized in that** the drug is useful in "Chemodynamic Therapy" or useful in "Radiochemodynamic Therapy" or "Radiodynamic Therapy" to be carried out by a radioactive ray, the drug has action mechanism as follows:

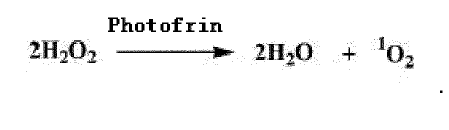

2. The drug according to claim 1, **characterized in that** the photofrin is a catalyst, which catalyzes the decomposition reaction of H₂O₂ to generate ¹O₂, does not directly participate the reaction and does not form a new compound.

3. The drug according to claim 1 or 2, **characterized in that** the photofrin is activated by a physical and/or chemical method to start catalyzing the decomposition of H₂O₂ to generate ¹O₂, which structure comprises all photofrins or precursors for synthesizing photofrins.

4. The drug according to claim 1 or 2, **characterized in that** in the reaction where the photofrin catalyzes the decomposition of H₂O₂ to generate ¹O₂, the chemical structure of the photofrin is not destroyed.

5. The drug according to claim 1 or 2, **characterized in that** under certain physical or chemical conditions, in the research of reaction of catalytically decomposing H₂O₂ to generate ¹O₂, it is able to determine the catalytic properties and catalytic efficiency of photofrin, and the research is merely used for screening a photofrin useful as an efficient catalyst for decomposition of H₂O₂ to generate ¹O₂.

6. The drug according to any one of claims 1 to 3, **characterized in that** under certain physical and chemical conditions, it can be used in combination with only a designated photofrin, and acts as an agonist of the reaction of decomposing H₂O₂ catalyzed by the designated photofrin to generate ¹O₂.

7. The drug according to any one of claims 1 to 3, **characterized in that** the certain physical and/or chemical conditions are electron beam, x-ray, r-ray, ion beam or other physical methods, or are protein binding or other chemical methods.

8. The drug according to claim 1, 3, 5 or 6, **characterized in that** the certain physical and/or chemical are used in combination with only the designated photofrin, used for rapidly starting or stopping the reaction of decomposing H₂O₂ to generate ¹O₂.

9. The drug according to any one of claims 1 to 8, **characterized in that** H₂O₂ or a preparation containing H₂O₂, is only used as a substrate of chemical reaction.

10. The drug according to claim 1, **characterized in that** in the "Chemodynamic Therapy", by the catalysis of the photofrin, a chemical method is used to start the in vivo decomposition of H₂O₂ to generate ¹O₂ for treatment of diseases, visible light and oxygen are not needed for the treatment, and its mechanism is totally different from that of photodynamic therapy.

11. The drug according to claim 1, **characterized in that** in the "Radiochemodynamic Therapy", a method uses electron beam, x-ray, r-ray, ion beam to activate the photofrin in vivo, to start or stop the decomposition of H₂O₂ to generate ¹O₂ for treatment of diseases, visible light and oxygen are not needed for the treatment, and its mechanism is totally different from that of photodynamic therapy, and from radiotherapy.

12. The drug according to claim 1, 10 or 11, **characterized in that** the "Chemodynamic Therapy" and "Radiochemodynamic Therapy" meet clinical requirements, that is, the reaction of decomposing H₂O₂ to generate ¹O₂ must occur in a site with a pathological change; the reaction of decomposing H₂O₂ to generate ¹O₂ must be rapid and highly efficient; the reaction of decomposing H₂O₂ to generate ¹O₂ must be controllable; and the reaction of decomposing H₂O₂ to generate ¹O₂ must be quantitative.

13. The drug according to claim 1, **characterized in that**: it is used in RCDT and/or CDT and/or RDT for treatment of a tumor.

14. The drug according to claim 1, **characterized in that**: it is used in RCDT and/or CDT and/or RDT for treatment of a tumor vessel.

15. The drug according to claim 1, **characterized in that**: it is used in RCDT for treatment of a vascular plaque.

16. The drug according to claim 1, **characterized in that**: it is used in RCDT for treatment of a topical intractable inflammation, ophthalmic disease, vascular tumor in organ, prostatic hyperplasia, and other benign diseases.

17. The drug according to claim 1, **characterized in that**: it is used in CDT for treatment of all skin diseases that can be treated by photodynamic therapy.

18. The drug according to claim 1, **characterized in that**: it is used in RCDT and/or CDT and/or RDT for treatment of a disease that cannot be efficiently treated by other therapeutical methods.

19. The drug according to claim 1, **characterized in that**: it is used in RCDT and/or CDT and/or RDT for treatment of all other diseases that can be treated by photodynamic therapy.
